# EUROPEAN PATENT APPLICATION

(11) **EP 4 811 366 A1**
(43) Date of publication of application: **23.09.2026**
(21) Application number: 24891004.4
(22) Date of filing: 05.07.2024
(51) Int. Cl.: G16C 60/00, G06N 20/00

(54) **PREDICTION METHOD AND PREDICTION SYSTEM**

(30) Priority: 16.11.2023 JP 2023195126
(71) Applicant: GC MFG Corporation, Shizuoka 410-1307 (JP)
(72) Inventor: HOKII, Yusuke, Tokyo 174-8585 (JP); YAMAMOTO, Koji, Tokyo 174-8585 (JP); AKIYAMA, Shigenori, Tokyo 174-8585 (JP)
(74) Representative: Müller-Boré & Partner Patentanwälte PartG mbB
(86) International application number: PCT/JP2024/024389
(87) International publication number: WO 2025/104959

(57) **Abstract**

To design a material required to satisfy a plurality of properties efficiently. A method performed by a prediction system according to an embodiment of the present invention includes acquiring a composition and a manufacturing condition of a material of interest; and predicting a plurality of properties of the material of interest by inputting the acquired composition and manufacturing condition of the material of interest into a machine learning model for predicting properties of a material based on its composition and manufacturing condition. The machine learning model is based on a mixture of continuous probability distributions.

## Description

### TECHNICAL FIELD

The present invention relates to a prediction method and a prediction system.

### BACKGROUND ART

Conventionally, in the development of materials, a method has been employed in which a prototype of a material is actually fabricated and the properties of the material are evaluated. In recent years, a technique known as materials informatics, which predicts material properties using machine learning, has also been employed.

### RELATED ART DOCUMENTS

### PATENT DOCUMENT

Patent Document 1: Japanese Unexamined Patent Application Publication No. 2021-111360

### SUMMARY OF THE INVENTION

### PROBLEMS TO BE SOLVED BY THE INVENTION

For example, glass ceramics for dental use must satisfy a plurality of properties related to color and translucency, and it is desired to be able to efficiently design a material required to satisfy a plurality of properties.

Therefore, it is an object of the present invention to efficiently design a material required to satisfy a plurality of properties.

### MEANS FOR SOLVING THE PROBLEMS

A method according to one embodiment of the present invention is a method performed by a prediction system, the method including acquiring a composition and a manufacturing condition of a material of interest; and predicting a plurality of properties of the material of interest by inputting the acquired composition and manufacturing condition of the material of interest into a machine learning model for predicting properties of a material based on its composition and manufacturing condition. The machine learning model is based on a mixture of continuous probability distributions.

### EFFECTS OF THE INVENTION

According to the present invention, it is possible to efficiently design a material required to satisfy a plurality of properties.

### BRIEF DESCRIPTION OF THE DRAWINGS

[FIG. 1] FIG. 1 is a diagram illustrating an overall configuration according to an embodiment of the present invention.
[FIG. 2] FIG. 2 is a functional block diagram of a prediction device according to the embodiment of the present invention.
[FIG. 3] FIG. 3 is a functional block diagram of a training device according to the embodiment of the present invention.
[FIG. 4] FIG. 4 is a flowchart of a prediction process for a plurality of properties according to the embodiment of the present invention.
[FIG. 5] FIG. 5 is a flowchart of a process of predicting a composition and a manufacturing condition according to the embodiment of the present invention.
[FIG. 6] FIG. 6 is a flowchart of a training process according to an embodiment of the present invention.
[FIG. 7] FIG. 7 is a table illustrating comparison results of a Student's t mixture regression model and a Gaussian mixture regression model according to the embodiment of the present invention.
[FIG. 8] FIG. 8 is of graphs illustrating comparison results of the Student's t mixture regression model and the Gaussian mixture regression model according to an embodiment of the present invention.
[FIG. 9] FIG. 9 is a flowchart of an empirical evaluation procedure for the Student's t mixture regression model according to the embodiment of the present invention.
[FIG. 10] FIG. 10 is of graphs illustrating comparison results of target values and experiment values according to the embodiment of the present invention.
[FIG. 11] FIG. 11 is of graphs illustrating comparison results of predicted values and experiment values according to the embodiment of the present invention.
[FIG. 12] FIG. 12 is a diagram illustrating an example of a screen in which material properties are visualized according to the embodiment of the present invention.
[FIG. 13] FIG. 13 is a diagram illustrating an example of a screen in which material properties are visualized according to the embodiment of the present invention.
[FIG. 14] FIG. 14 is a diagram for explaining designation of material properties on a screen on which material properties are visualized according to the embodiment of the present invention.
[FIG. 15] FIG. 15 is a hardware configuration diagram of a prediction device and a training device according to the embodiment of the present invention.

### DESCRIPTION OF EMBODIMENTS

Embodiments of the present invention will be described below with reference to the drawings.

### <Explanation of Terms>

- As used herein, a "material" may refer to any freely chosen material. For example, a "material" is a medical material (e.g., dental materials). For example, a "material" is any one of a ceramic, glass, glass ceramic, polymeric material, composite resin, glass ionomer, and metal (e.g., dental ceramics, dental glass-ceramics, dental polymeric materials, dental composite resins, dental glass ionomers, dental metals, dental impression materials).
- As used herein, a "property" may refer to any freely chosen property. For example, a "property" is a color and translucency property of a material. For example, a color and translucency property of a material may include one or more of the following: a CIE LAB color system value (for example, L*, a*, or b* values), total light transmittance (TT), collimated light component (PT), diffuse (scattered) light component (DIF), translucency parameter (TP), haze, or contrast ratio (CR). In other words, a material's color and translucency property may be represented by all or a subset of these values.

### <Overall Configuration>

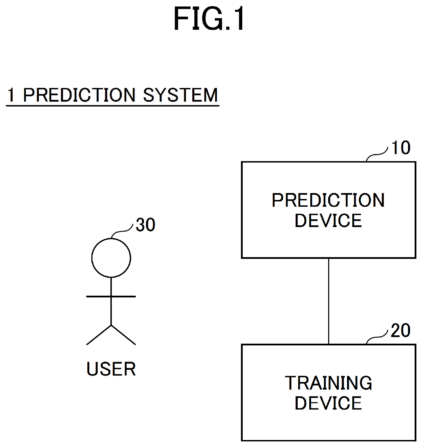

FIG. 1 is a diagram illustrating an overall configuration according to an embodiment of the present invention. A user 30 operates a prediction device 10 and a training device 20. Although the prediction device 10 and the training device 20 are described as separate devices in FIG. 1, the prediction device 10 and the training device 20 may be implemented as one device.

### <<Prediction Device>>

The prediction device 10 is a device for predicting a plurality of properties of a material and a composition and a manufacturing condition (for example, a heat treatment condition (e.g., a heating rate, a holding temperature, holding time, a cooling rate, and cooling time)) of a material. The prediction device 10 includes one or more computers. The prediction device 10 can send and receive data to and from the training device 20 via a freely chosen network.

Specifically, the prediction device 10 can predict a plurality of properties of a material of interest based on its composition and manufacturing condition. The prediction device 10 can also predict a composition and a manufacturing condition of a material of interest based on a plurality of properties of the material of interest. The device for predicting a plurality of material properties and the device for predicting a material composition and a material manufacturing condition may be the same device or may be different devices.

### <<Training device>>

The training device 20 is a device for generating a machine learning model for predicting a plurality of properties of a material based on its composition and manufacturing condition. The training device 20 is composed of one or more computers. The training device 20 can send and receive data to and from the prediction device 10 via a freely chosen network.

### <<Functional Blocks>

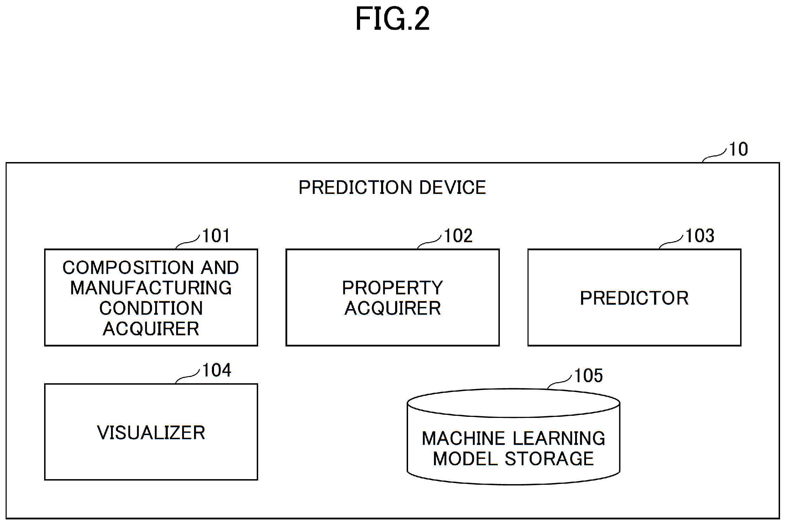

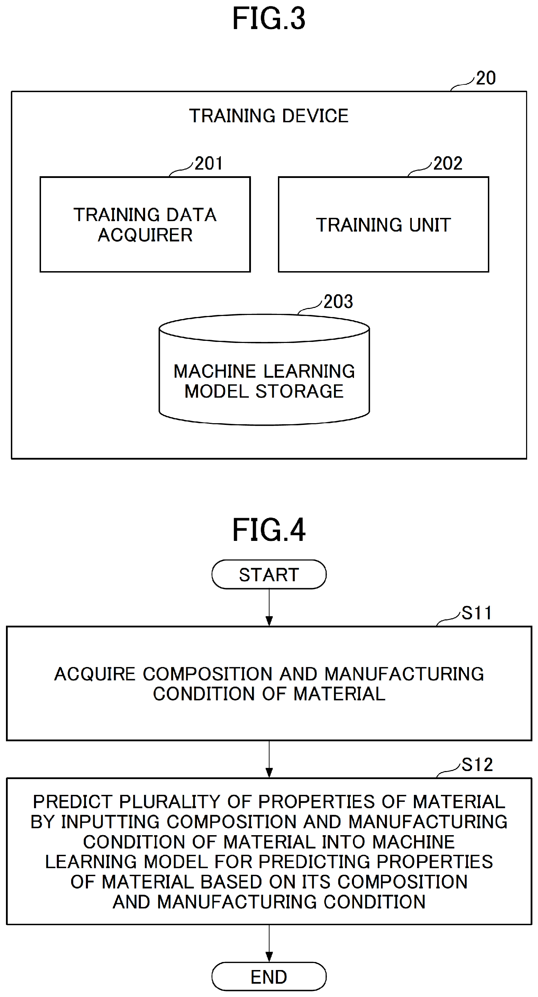

Hereinafter, functional blocks of the prediction device 10 will be described with reference to FIG. 2, and functional blocks of the training device 20 will be described with reference to FIG. 3.

### [Prediction Device]

FIG. 2 is a functional block diagram of the prediction device 10 according to the embodiment of the present invention. The prediction device 10 may include a composition and manufacturing condition acquirer 101, a property acquirer 102, a predictor 103, a visualizer 104, and a machine learning model storage 105. The prediction device 10 may function as the composition and manufacturing condition acquirer 101, the property acquirer 102, the predictor 103, and the visualizer 104, by executing a program.

The composition and manufacturing condition acquirer (also referred to simply as the "acquirer") 101 acquires a composition and a manufacturing condition of a material. For example, the composition and manufacturing condition acquirer 101 acquires a composition and a manufacturing condition of a material that are input by the user 30 to the prediction device 10 or the like.

The property acquirer (also referred to simply as the "acquirer") 102 acquires a plurality of properties of a material. For example, the property acquirer 102 acquires a plurality of properties of a material that are input by the user 30 to the prediction device 10 or the like.

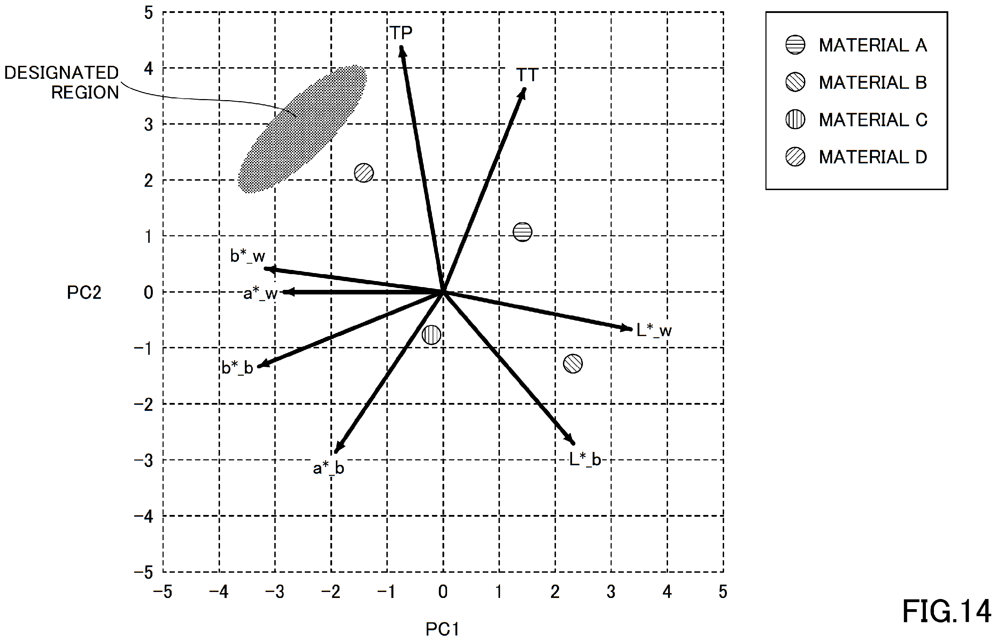

The property acquirer 102 may acquire a plurality of properties of a material by acquiring a designation of a region in a space representing a plurality of properties of materials (for example, designating a region in the screen as illustrated in FIG. 14) and specifying a plurality of properties of a material corresponding to the region.

### [Properties of Material]

Hereinafter, properties of a material will be described. A plurality of properties of a material may be a plurality of parameters (e.g., hue, lightness, and saturation) for determining one property (e.g., color) of a material, or a plurality of parameters (e.g., a parameter for determining color and a parameter for determining translucency) for determining a plurality of properties (e.g., color and translucency) of a material.

For example, "properties" are properties related to color and translucency of a material. For example, there are eight properties related to color and translucency of a material: six CIE LAB color system values (L*, a*, and b* values when the material background is white, and L*, a*, and b* values when the material background is black), a total light transmittance (TT value), and a translucency parameter (TP value).

The predictor 103 predicts a plurality of properties of a material of interest and a composition and a manufacturing condition of the material of interest. The predictor 103 may output the predicted plurality of properties of the material and the predicted composition and manufacturing condition of the material, for example, by displaying them on the prediction device 10 or transmitting them to other devices.

Detailed descriptions will be given in [Prediction of Plurality of Properties of Material] and [Prediction of Composition and Manufacturing Condition of Material] below.

### [Prediction of Plurality of Properties of Material]

The predictor 103 inputs a composition and a manufacturing condition of a material of interest acquired by the composition and manufacturing condition acquirer 101 into a machine learning model (specifically, a machine learning model for predicting a plurality of properties of a material based on its composition and manufacturing condition) stored in the machine learning model storage 105, and predicts a plurality of properties of the material of interest.

In this way, a plurality of properties that are acquired without actually fabricating a prototype material (in other words, predicted by the prediction device 10), of a material to be manufactured by a composition and a manufacturing condition designated by the user 30 can be used for material design.

### [Prediction of Composition and Manufacturing Condition of Material]

The predictor 103 predicts a composition and a manufacturing condition of a material of interest based on a plurality of properties of the material of interest, which are acquired by the property acquirer 102, by performing inverse analysis using a machine learning model stored in the machine learning model storage 105 (specifically, a machine learning model for predicting a plurality of properties of a material based on its composition and manufacturing condition).

In this way, a composition and a manufacturing condition of a material for realizing a plurality of properties designated by the user 30 and acquired without actually fabricating a prototype material (in other words, predicted by the prediction device 10) can be used for material design.

### [Inverse Analysis Using Machine Learning Model]

Hereinafter, inverse analysis using a machine learning model will be described. In the inverse analysis using a machine learning model (specifically, a machine learning model for predicting a plurality of properties of a material based on its composition and manufacturing condition), a composition and a manufacturing condition (i.e., values that are input to the machine learning model) that satisfy a plurality of properties of a material of interest are searched.

The visualizer 104 plots a plurality of materials within a space representing a plurality of material properties, thereby visualizing a plurality of properties of each material. The space is defined by a reduced number of dimensions obtained through dimensionality reduction (for example, principal component analysis) applied to a plurality of material properties (for example, eight properties). The resulting space may be a two-dimensional space or a three-dimensional space.

The machine learning model storage 105 stores a machine learning model generated by the training device 20 (specifically, a machine learning model for predicting a plurality of properties of a material based on its composition and manufacturing condition).

### [Training Device]

FIG. 3 is a functional block diagram of the training device 20 according to the embodiment of the present invention. The training device 20 includes a training data acquirer 201, a training unit 202, and a machine learning model storage 203. The training device 20 functions as the training data acquirer 201 and the training unit 202 by executing a program.

The training data acquirer 201 acquires training data to be used when generating a machine learning model (i.e., a machine learning model for predicting a plurality of properties of a material based on its composition and manufacturing condition). Specifically, the training data acquirer 201 acquires a composition and a manufacturing condition of a material, and a plurality of properties, namely measured values, of a material actually manufactured (prototype fabrication, etc.) by the composition and manufacturing condition.

The training unit 202 performs machine learning using the training data acquired by the training data acquirer 201 to generate a machine learning model for predicting a plurality of properties of a material based on its composition and manufacturing condition.

Specifically, when a composition and a manufacturing condition of a material and a plurality of properties of the material are input to the model, the training unit 202 estimates parameters by an EM algorithm (expectation maximization algorithm) based on the input data. More specifically, the model is trained by estimating each parameter so that a log-likelihood function is maximized.

The machine learning model storage 203 stores a machine learning model generated by the training unit 202 (specifically, a machine learning model for predicting a plurality of properties of a material based on its composition and manufacturing condition).

### <Machine Learning Model>

The machine learning model will be described hereinafter. In the present invention, a method of a mixture model is used. The machine learning model used in the present invention is based on a mixture of continuous probability distributions (specifically, a regression model in which multiple multidimensional (multivariate) continuous probability distributions are mixed). In other words, in the machine learning model used in the present invention, continuous probability distributions each having the same number of dimensions as the number of properties of a material are mixed in accordance with the number of clusters when data of the properties are clustered. As a result, a probability density distribution of the data is obtained, which is used as a regression model.

Hereinafter, as examples of regression models in which a plurality of multidimensional continuous probability distributions are mixed, a Gaussian mixture regression model (hereinafter also referred to as "GMR") and a Student's t mixture regression model (hereinafter also referred to as "SMR") will be described. As will be described later, the Student's t mixture regression model had higher prediction accuracy than the Gaussian mixture regression model.

The following illustrates a multidimensional Gaussian distribution (normal distribution) and its mixture.$\begin{matrix}N \left(x\left|μ, Σ\right.\right) = {\left(2π\right)}^{- \frac{D}{2}} {\left|Σ\right|}^{- \frac{1}{2}} exp \left\{-\frac{1}{2}{\left(x-μ\right)}^{T}{Σ}^{- 1}\left(x-μ\right)\right\} \\ p \left(x\right) = {\sum }_{k = 1}^{K} {π}_{k} N \left(x\left|{μ}_{k}, {Σ}_{k}\right.\right)\end{matrix}$

The following illustrates a multidimensional Student's t distribution and its mixture.$\begin{matrix}St \left(x\left|μ, Λ, v\right.\right) = \frac{Γ \left(v/2+d/2\right) {\left|Λ\right|}^{1 / 2}}{Γ \left(v/2\right) {\left(vπ\right)}^{d / 2}} {\left(1+\frac{{Δ}^{2}}{v}\right)}^{- \left(v+d\right) / 2} \\ Γ \left(t\right) = {\int }_{0}^{∞} {z}^{t - 1} {e}^{- z} dz Γ function \\ {Δ}^{2} = {\left(x-μ\right)}^{T} Λ \left(x-μ\right) squared Mahalanobis distance \\ p \left(x\left|μ, Λ, v, π\right.\right) = {\sum }_{k = 1}^{K} {π}_{k} St \left(x\left|{μ}_{k}, {Λ}_{k}, {v}_{k}\right.\right)\end{matrix}$
µ: mean vector
Σ: covariance matrix
A: precision matrix
v: degrees of freedom

The mixing ratios of the multidimensional Gaussian distribution and the multidimensional Student's t distribution are as follows.${\sum }_{k = 1}^{K} {π}_{k} = 1 0 \leq {π}_{k} \leq 1$

The parameters and hyperparameters in a machine learning model are as follows.
- SMR
   Parameters: µ, A, π, v (degrees of freedom)
   Hyperparameters: number of Student-t distributions, type of precision matrix (A)
- GMR
   Parameters: µ, Σ, π
   Hyperparameters: number of normal distributions, type of covariance matrix (Σ)

### <Processing Method>

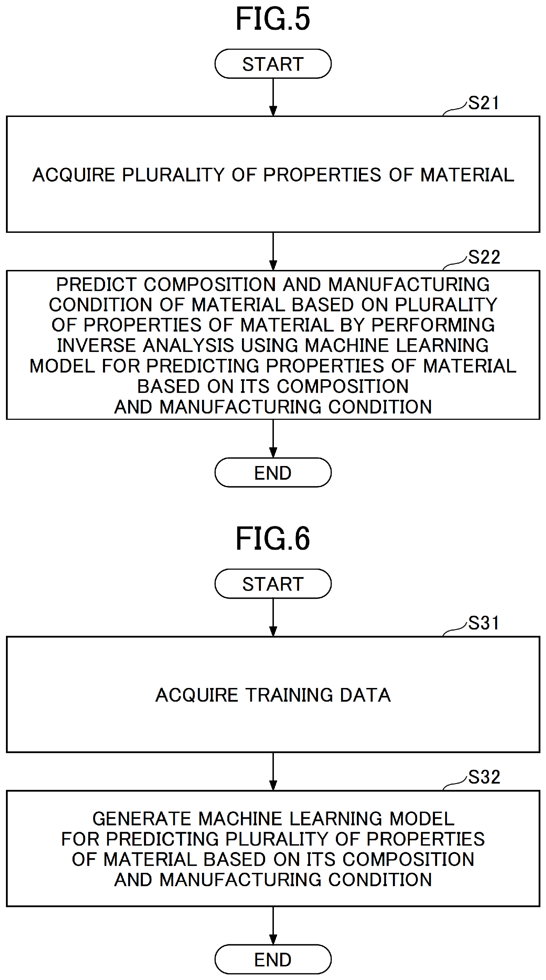

Hereinafter, a prediction process for a plurality of properties will be described with reference to FIG. 4. A prediction process for a composition and a manufacturing condition will also be described with reference to FIG. 5, and a training process will be described with reference to FIG. 6.

FIG. 4 is a flowchart of a prediction process for a plurality of properties according to the embodiment of the present invention.

In step 11 (S11), the composition and manufacturing condition acquirer 101 of the prediction device 10 acquires a composition and a manufacturing condition of a material of interest.

In step 12 (S12), the predictor 103 of the prediction device 10 inputs the composition and manufacturing condition of the material of interest acquired in step 11 to a machine learning model for predicting a plurality of properties of a material based its composition and manufacturing condition, thereby predicting a plurality of properties of the material of interest.

Thereafter, the predictor 103 of the prediction device 10 may output the predicted properties of the material of interest predicted in step 12, for example by displaying them on the prediction device 10 or transmitting them to other devices.

FIG. 5 is a flowchart of a prediction process for a composition and a manufacturing condition according to the embodiment of the present invention.

In step 21 (S21), the property acquirer 102 of the prediction device 10 acquires a plurality of properties of a material of interest.

In step 22 (S22), the predictor 103 of the prediction device 10 predicts a composition and a manufacturing condition of the material of interest based on the plurality of properties of the material of interest acquired in S21 by performing inverse analysis using a machine learning model for predicting a plurality of properties of a material based on its composition and manufacturing condition.

Thereafter, the predictor 103 of the prediction device 10 may output the composition and manufacturing condition of the material of interest predicted in S22, for example, by displaying them on the prediction device 10 or transmitting them to other devices.

FIG. 6 is a flowchart of a training process according to the embodiment of the present invention.

In step 31 (S31), the training data acquirer 201 of the training device 20 acquires training data to be used when generating a machine learning model (specifically, a machine learning model for predicting a plurality of properties of a material based on its composition and manufacturing condition).

In step 32 (S32), the training unit 202 of the training device 20 performs machine learning using the training data acquired in step 31 to generate a machine learning model for predicting a plurality of properties of a material based on its composition and manufacturing condition.

### <Comparison of SMR and GMR>

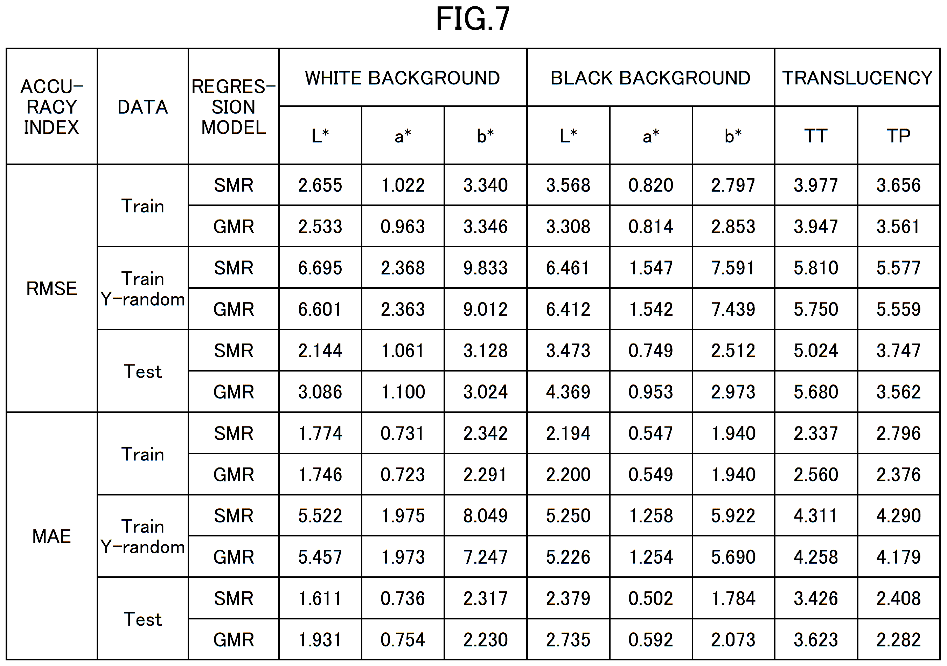

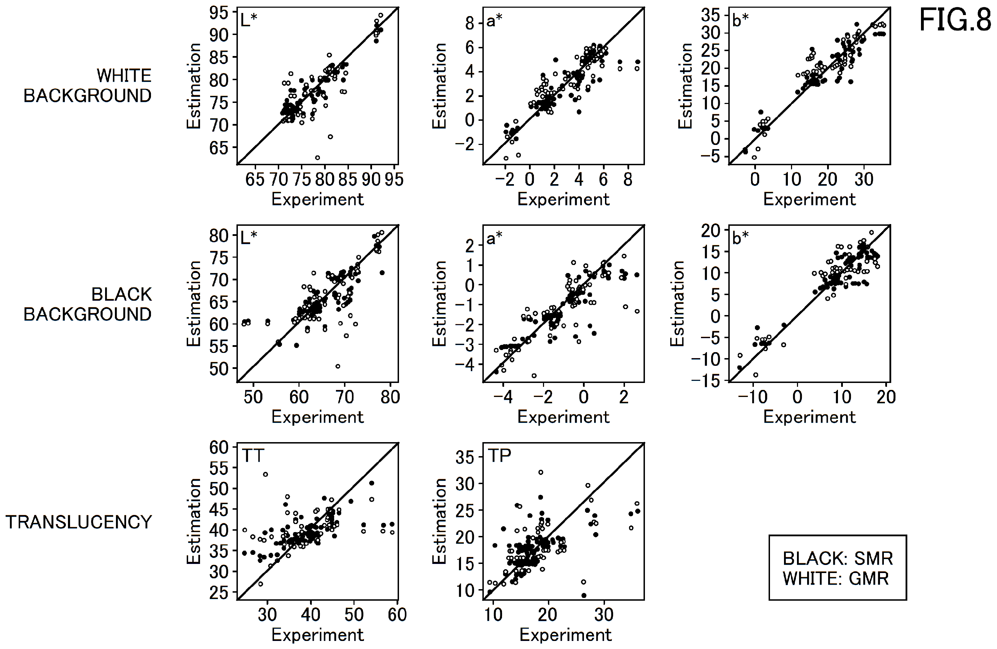

Hereinafter, a comparison result between a Student's t mixture regression model (SMR) and a mixed Gaussian mixture regression model (GMR) will be described with reference to FIGS. 7 and 8.

FIG. 7 is a table illustrating comparison results between a Student's t mixture regression model (SMR) and a Gaussian mixture regression model (GMR) according to the embodiment of the present invention. FIG. 7 illustrates the values of prediction accuracy (using two accuracy indices, a root mean squared error (RMSE) and a mean absolute error (MAE)) of the SMR and the GMR.

Specifically, the values of the respective accuracy indices when predicting the L* value of a white background, the a* value of a white background, the b* value of a white background, the L* value of a black background, the a* value of a black background, the b* value of a black background, the TT value, and the TP value, which are the properties of the material, are illustrated. The data of "Train" indicates a case of prediction using the training data; the data of "Train Y-random" indicates a case of prediction using data obtained by randomly combining input data and output data of the training data; and the data of "Test" indicates a case of prediction using the test data.

As illustrated in FIG. 7, the prediction accuracy of the Student's t mixture regression model (SMR) was higher than that of the Gaussian mixture regression model (GMR) in both cases of an RMSE and an MAE.

FIG. 8 is of graphs illustrating comparison results between the mixed Student's t mixture regression model (SMR) and the Gaussian mixture regression model (GMR) according to the embodiment of the present invention. FIG. 8 illustrates scatter plots of predicted values (the values obtained by the prediction of the prediction device 10, "Prediction") and experimental values (the values obtained in the experiment, "Experiment") of the SMR and the GMR.

Specifically, FIG. 8 illustrates scatter plots of the predicted value (Prediction) and the experimental value (Experiment) of the L* value of a white background, the a* value of a white background, the b* value of a white background, the L* value of a black background, the a* value of a black background, the b* value of a black background, the TT value, and the TP value, which are properties of the material.

As illustrated in FIG. 8, the degree of agreement between the predicted values (Prediction) and the experimental values (Experiment) of the Student's t mixture regression model (SMR) was higher than that of the Gaussian mixture regression model (GMR).

Therefore, it is preferable to use the Student's t mixture regression model (SMR) with high prediction accuracy. The SMR using the t distribution can not only cope with data dispersion, but also is robust to data with many outliers (noise) because the distribution has a thick base.

### [Empirical Evaluation]

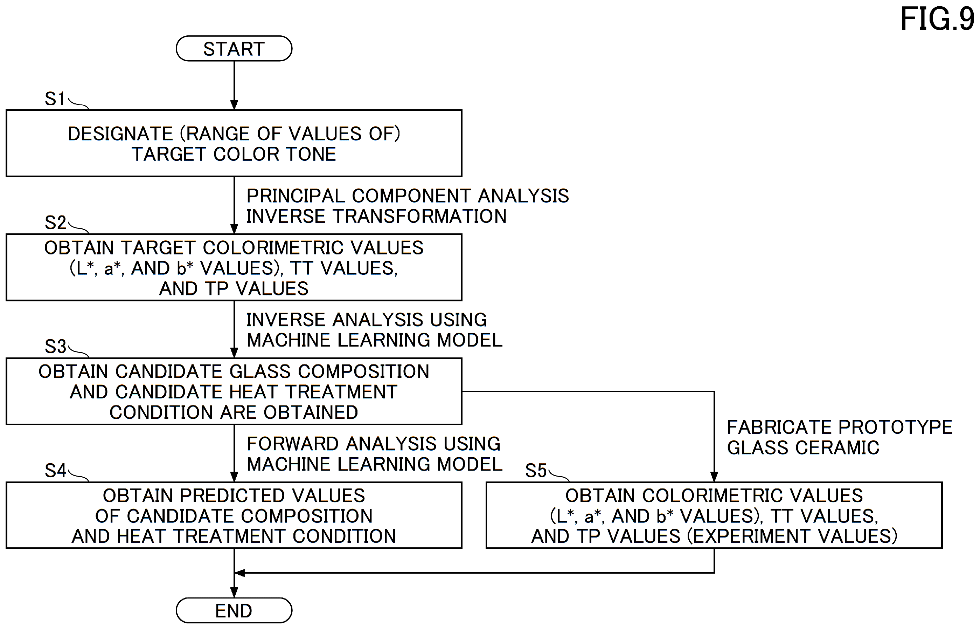

FIG. 9 is a flowchart of an empirical evaluation procedure of the Student's t evaluation regression model (SMR) according to the embodiment of the present invention. In the procedure, the following variables are used.
- Explanatory variables: glass composition, heat treatment condition
- Objective variables: L* values, a* values, b* values, TT values, and TP values of a white background and a black background

In step 1 (S1), a target color tone is designated (for example in a screen as illustrated in FIG. 14).

In step 2 (S2), the target color tone (value range) in S1 is subjected to principal component analysis inverse transformation to obtain target values of properties (specifically, target colorimetric values (L*, a*, and b* values), TT values, and TP values) of a material.

In step 3 (S3), a candidate glass composition and a candidate heat treatment condition are obtained by inverse analysis of the target values obtained in S2 using a machine learning model.

In step 4 (S4), predicted values of the properties of the material are obtained by forward analysis of the candidate glass composition and candidate heat treatment condition obtained in S3 using a machine learning model.

In step 5 (S5), a prototype glass ceramic is fabricated by the candidate glass composition and candidate heat treatment condition obtained in S3, and experimental values of colorimetric values (L*, a*, and b* values), a TT value, and a TP value of the prototype are obtained.

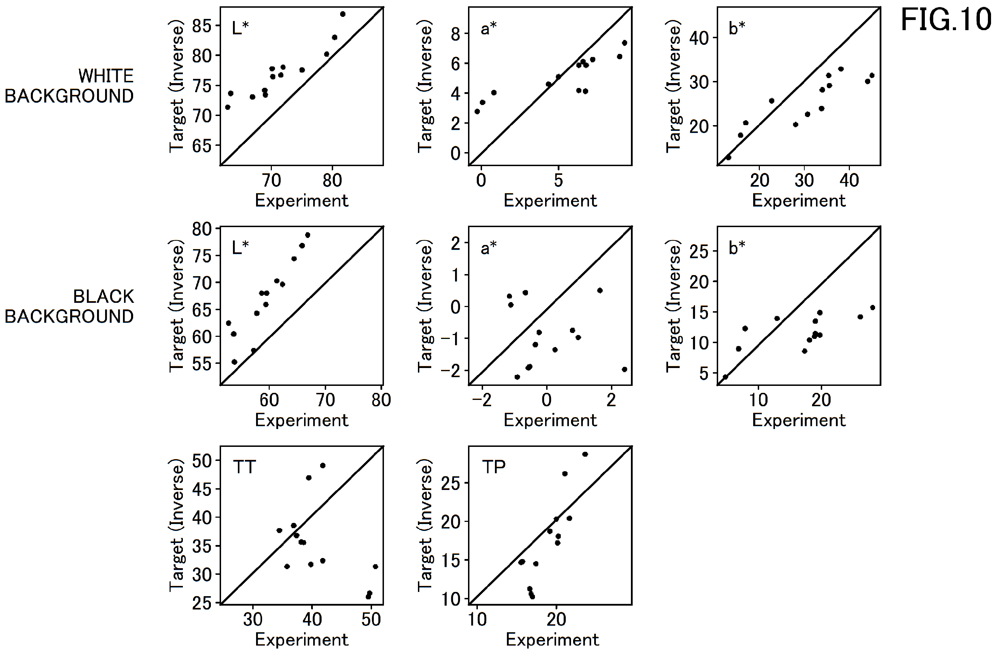

FIG. 10 illustrates comparison results between the target values and the experimental values according to the embodiment of the present invention. FIG. 10 illustrates scatter plots of the target values (target values in S2 of FIG. 9, "Target") and the experimental values (experimental values in S5 of FIG. 9, "Experiment") in the empirical evaluation of the Student's t mixture regression model (SMR) of FIG. 9.

Specifically, scatter plots of the target values (target values in S2 of FIG. 9, "Target") and the experimental values (experimental values in S5 of FIG. 9, "Experiment") of the L* value of a white background, the a* value of a white background, the b* value of a white background, the L* value of a black background, the a* value of a black background, the b* value of a black background, the TT value, and the TP value, which are properties of the material, are illustrated.

As illustrated in FIG. 10, the target values and the experimental values are generally the same in all cases.

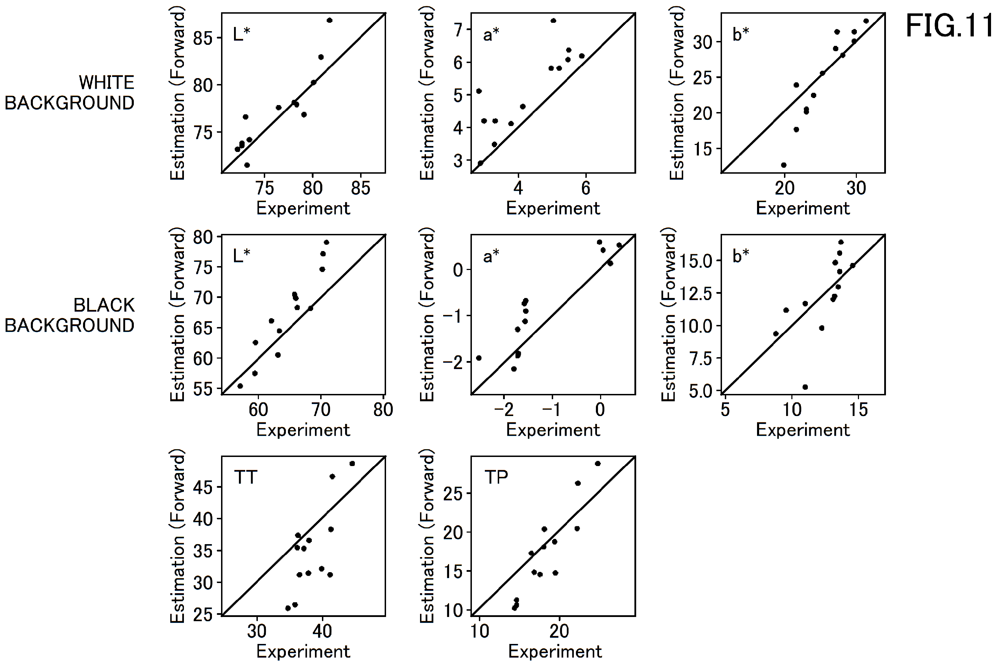

FIG. 11 illustrates comparison results between the predicted values and the experimental values according to the embodiment of the present invention. FIG. 11 illustrates scatter plots of the predicted values (predicted values in S4 of FIG. 9, "Prediction") and the experimental values (experimental values in S5 of FIG. 9, "Experiment") in the empirical evaluation of the mixed Student's t mixture regression model (SMR) of FIG. 9.

Specifically, scatter plots of the predicted values (predicted values in S4 of FIG. 9, "Prediction") and the experimental values (experimental values in S5 of FIG. 9, "Experiment") of the L* value of a white background, the a* value of a white background, the b* value of a white background, the L* value of a black background, the a* value of a black background, the b* value of a black background, the TT value, and the TP value, which are properties of the material, are illustrated.

As illustrated in FIG. 11, the predicted values are generally the same as the experimental values.

The experimental values of properties of a material obtained by fabricating a prototype using the candidate glass composition and candidate heat treatment condition obtained in S3, together with data consisting of the candidate glass composition and candidate heat treatment condition obtained in S3, are added to the training data acquirer 201 as training data and used for training the machine learning model. Thus, the performance of the machine learning model can be improved.

### <Visualization of Properties of Material>

Visualization of properties of a material will be described hereinafter. The prediction device 10 is capable of visualizing at least one of a material property of a prototype that has already been fabricated or a material property predicted by the prediction device 10.

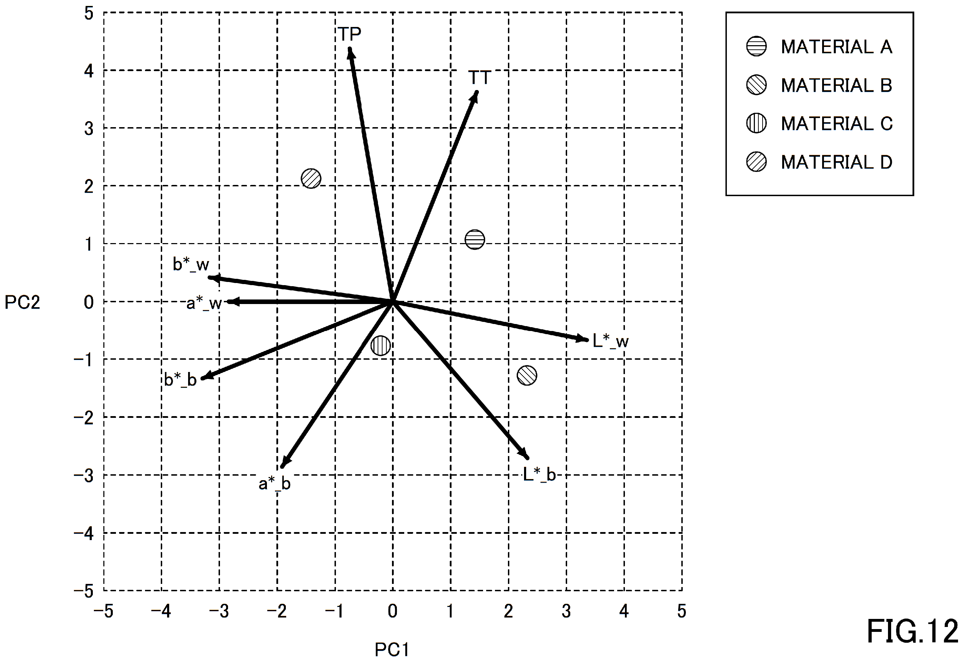

FIG. 12 illustrates an example of a screen in which material properties are visualized according to the embodiment of the present invention.

The prediction device 10 generates a space representing a plurality of material properties by dimensionally reducing these properties using principal component analysis. In this space, the first and second principal components (PC1 and PC2, as illustrated in FIG. 12) define the horizontal and vertical axes. The plurality of material properties are, as illustrated in FIG. 12, the L* value of a white background, the a* value of a white background, the b* value of a white background, the L* value of a black background, the a* value of a black background, the b* value of a black background, the TT value, and the TP value. Then, the prediction device 10 plots each material (materials A to D, as illustrated in FIG. 12) in the space representing a plurality of material properties. In other words, a point representing each material (materials A to D, as illustrated in FIG. 12) is plotted on the space.

In FIG. 12, the arrow of L*_w is the axis of the L* value of a white background (i.e., the arrow indicates the direction in which the L* value of a white background increases); the arrow of a*_w is the axis of the a* value of a white background (i.e., the arrow indicates the direction in which the a* value on a white background increases); the arrow of b*_w is the axis of the b* value of a white background (i.e., the arrow indicates the direction in which the b* value on a white background increases); the arrow of L*_b is the axis of the L* value of a black background (i.e., the arrow indicates the direction in which the L* value of a black background increases); the arrow of a*_b is the axis of the a* value of a black background (i.e., the arrow indicates the direction in which the a* value of a black background increases); the arrow of b*_b is the axis of the b* value of a black background (i.e., the arrow indicates the direction in which the b* value of a black background increases); the arrow of TT is the axis of the TT value (i.e., the arrow indicates the direction in which the TT value increases); and the arrow of TP is the axis of the TP value (i.e., the arrow indicates the direction in which the TP value increases).

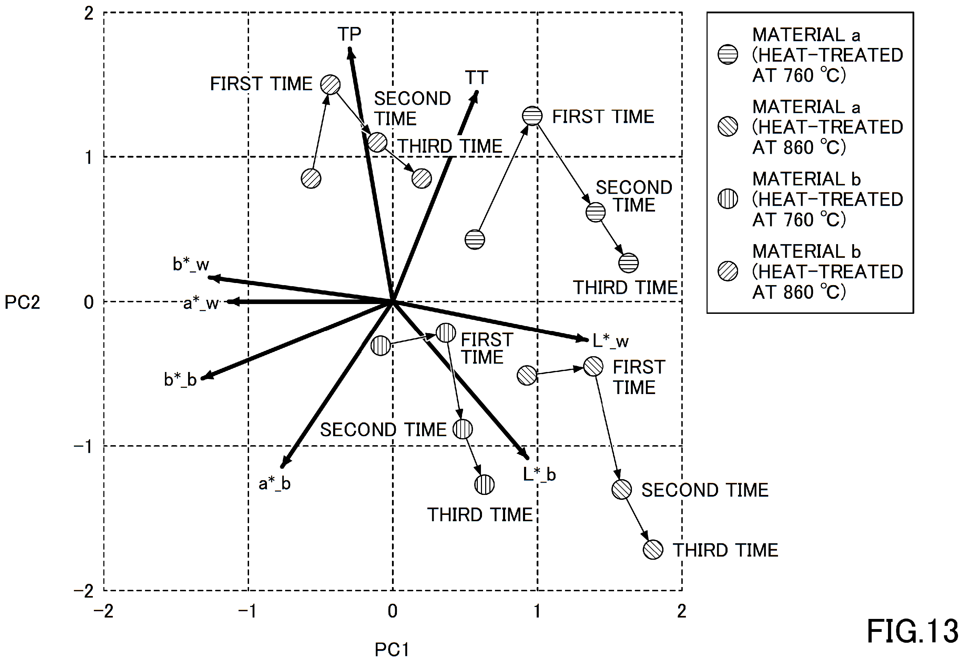

FIG. 13 is an example of a screen in which material properties are visualized according to the embodiment of the present invention.

Similarly to FIG. 12, the prediction device 10 generates a space representing a plurality of material properties by dimensionally reducing these properties using principal component analysis. In this space, the first and second principal components (PC1 and PC2, as illustrated in FIG. 13) define the horizontal and vertical axes. The plurality of material properties are, as illustrated in FIG. 13, the L* value of a white background, the a* value of a white background, the b* value of a white background, the L* value of a black background, the a* value of a black background, the b* value of a black background, the TT value, and the TP value. Then, the prediction device 10 plots each material (in the example of FIG. 13, material a (heat-treated at 760 °C), material a (heat-treated at 860 °C), material b (heat-treated at 760 °C), and material b (heat-treated at 860 °C)) in a space representing a plurality of material properties. In other words, a point representing each material (in the example of FIG. 13, material a (heat-treated at 760 °C), material a (heat-treated at 860 °C), material b (heat-treated at 760 °C), and material b (heat-treated at 860 °C)) is plotted on the space.

Similarly to FIG. 12, in FIG. 13, the arrow of L*_w is the axis of the L* value of a white background (i.e., the arrow indicates the direction in which the L* value of a white background increases); the arrow of a*_w is the axis of the a* value of a white background (i.e., the arrow indicates the direction in which the a* value on a white background increases); the arrow of b*_w is the axis of the b* value of a white background (i.e., the arrow indicates the direction in which the b* value on a white background increases); the arrow of L*_b is the axis of the L* value of a black background (i.e., the arrow indicates the direction in which the L* value of a black background increases); the arrow of a*_b is the axis of the a* value of a black background (i.e., the arrow indicates the direction in which the a* value of a black background increases); the arrow of b*_b is the axis of the b* value of a black background (i.e., the arrow indicates the direction in which the b* value of a black background increases); the arrow of TT is the axis of the TT value (i.e., the arrow indicates the direction in which the TT value increases); and the arrow of TP is the axis of the TP value (i.e., the arrow indicates the direction in which the TP value increases).

In FIG. 13, each material (material a (heat-treated at 760 °C), material a (heat-treated at 860 °C), material b (heat-treated at 760 °C), and material b (heat-treated at 860 °C)) is plotted at a position corresponding to the number of times the heat treatment has been performed. For example, points representing each material are plotted at property positions before the heat treatment, after the first heat treatment (in the example of FIG. 13, heat treatment at 760 °C or heat treatment at 860 °C), after the second heat treatment, and after the third heat treatment. In this way, it is possible to visualize the properties of the material subjected to heat treatment according to the manufacturing condition (for example, a heat treatment condition (for example, a heating rate, a holding temperature, holding time, a cooling rate, and cooling time.)).

FIG. 14 is a diagram for explaining designation of material properties on a screen on which material properties are visualized according to the embodiment of the present invention. The user 30 can designate properties on a screen on which the material properties displayed on the prediction device 10 or the like as illustrated in FIG. 14 are visualized. The prediction device 10 acquires the designation of a region on the screen as illustrated in FIG. 14 and designates a plurality of properties of a material corresponding to the region. More specifically, the prediction device 10 performs a principal component analysis inverse transform on the PC1 and PC2 of the designated region to calculate material property values corresponding to the designated region, including the L* value of a white background, the a* value of a white background, the b* value of a white background, the L* value of a black background, the a* value of a black background, the b* value of a black background, the TT value, and the TP value corresponding to the designated region.

### <Effects>

- According to one embodiment of the present invention, the user 30 can easily know a plurality of properties of a material manufactured by a composition and a manufacturing condition designated by the user 30.
- According to one embodiment of the present invention, the user 30 can easily know a composition and a manufacturing condition for realizing a plurality of properties of a material designated by the user 30. Furthermore, the user 30 can easily designate a plurality of desired properties on a screen in which properties of a material are visualized.

### <Hardware Configuration>

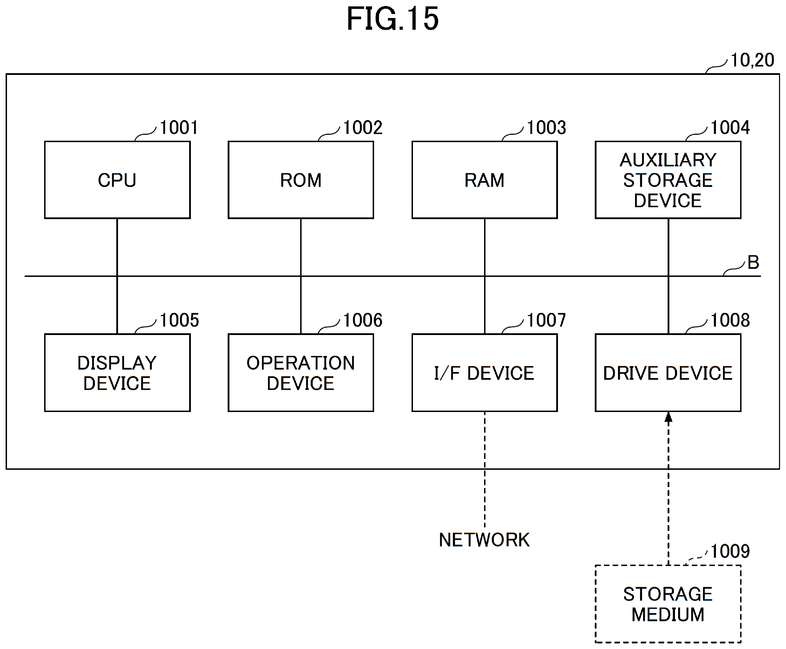

FIG. 15 is a hardware configuration diagram of the prediction device 10 and the training device 20 according to the embodiment of the present invention. The prediction device 10 and the training device 20 have a CPU (central processing unit) 1001, a ROM (read only memory) 1002, and a RAM (random access memory) 1003. The CPU 1001, the ROM 1002, and the RAM 1003 form a so-called computer. The prediction device 10 and the training device 20 can have an auxiliary storage device 1004, a display device 1005, an operation device 1006, an interface (I/F) device 1007, and a drive device 1008. The hardware of the prediction device 10 and the training device 20 are connected to each other via a bus B.

The CPU 1001 is an arithmetic device for executing various programs installed in the auxiliary storage device 1004. The CPU 1001 executes a program to perform the processes described herein.

The ROM 1002 is a nonvolatile memory. The ROM 1002 functions as a main storage device for storing various programs and data necessary for the CPU 1001 to execute various programs installed in the auxiliary storage device 1004. Specifically, the ROM 1002 functions as a main storage device for storing boot programs such as BIOS (basic input/output system) and EFI (extensible firmware interface).

The RAM 1003 is a volatile memory such as DRAM (dynamic random access memory) and SRAM (static random access memory). The RAM 1003 functions as a main storage device for providing a work area expanded when various programs installed in the auxiliary storage device 1004 are executed by the CPU 1001.

The auxiliary storage device 1004 is an auxiliary storage device for storing various programs and information used when various programs are executed.

The display device 1005 is a display device for displaying the internal state or the like of the prediction device 10 and the training device 20.

The operation device 1006 is an input device for an operator of the prediction device 10 and the training device 20 to input various instructions to the prediction device 10 and the training device 20.

The I/F device 1007 is a communication device for connecting to a network and communicating with other devices.

The drive device 1008 is a device for setting a storage medium 1009. Herein, the storage medium 1009 includes a medium for optically, electrically or magnetically recording information such as a CD (compact disk)-ROM, a flexible disk, a magneto-optical disk and the like. The storage medium 1009 may also include a semiconductor memory for electrically recording information such as a ROM, a flash memory and the like.

The various programs to be installed in the auxiliary storage device 1004 are installed by, for example, setting the distributed storage medium 1009 in the drive device 1008 and reading the various programs recorded in the storage medium 1009 by the drive device 1008. Alternatively, the various programs to be installed in the auxiliary storage device 1004 may be downloaded from a network via the I/F device 1007.

Although the embodiments of the present invention have been described in detail above, the present invention is not limited to the specific embodiments described above, and various modifications and alterations are possible within the scope of the present invention described in the appended claims.

This international application claims priority based on Japanese Patent Application No. 2023-195126 filed on November 16, 2023, and the entire contents of Japanese Patent Application No. 2023-195126 are hereby incorporated by reference.

### REFERENCE SIGNS LIST

- 1: Prediction system
- 10: Prediction device
- 20: Training device
- 30: User
- 101: Composition and manufacturing condition acquirer
- 102: Property acquirer
- 103: Predictor
- 104: Visualizer
- 105: Machine learning model storage
- 201: Training data acquirer
- 202: Training unit
- 203: Machine learning model storage
- 1001: CPU
- 1002: ROM
- 1003: RAM
- 1004: Auxiliary storage device
- 1005: Display device
- 1006: Operation device
- 1007: I/F device
- 1008: Drive device
- 1009: Storage medium

## Claims

1. A method performed by a prediction system, the method comprising:
acquiring a composition and a manufacturing condition of a material of interest; and
predicting a plurality of properties of the material of interest by inputting the acquired composition and manufacturing condition of the material of interest into a machine learning model for predicting properties of a material based on its composition and manufacturing condition, wherein
the machine learning model is based on a mixture of continuous probability distributions.

2. A method performed by a prediction system, the method comprising:
acquiring a plurality of properties of a material of interest; and
predicting a composition and a manufacturing condition of the material of interest based on the acquired plurality of properties of the material of interest by performing inverse analysis using a machine learning model for predicting properties of a material based on its composition and manufacturing condition, wherein
the machine learning model is based on a mixture of continuous probability distributions.

3. The method according to claim 2, further comprising:
visualizing the plurality of properties of the material of interest by plotting the material of interest in a space representing the plurality of properties of the material of interest, for each of materials of interest.

4. The method according to claim 3, wherein
the space is a space of dimensions after dimensionality reduction of the plurality of properties of the material of interest.

5. The method according to claim 3, wherein
the acquiring the plurality of properties of the material of interest is acquiring a designation of a region in the space to specify the plurality of properties of the material of interest corresponding to the region.

6. The method according to claim 1 or 2, wherein
the machine learning model is a Student's t mixture regression model.

7. The method according to claim 1 or 2, wherein
the plurality of properties are properties relating to color and translucency of the material of interest.

8. The method according to claim 1 or 2, wherein
the plurality of properties relating to color and translucency include at least one of a CIE LAB color system value, a total light transmittance, a collimated light component, a diffuse (scattered) light component, a Translucency Parameter (TP value), a haze, or a Contrast Ratio (CR value).

9. The method according to claim 1 or 2, wherein
the plurality of properties relating to color and translucency are an L* value, a* value, b* value, a total light transmittance, a collimated light component, a diffuse (scattered) light component, a Translucency Parameter (TP value), a haze, and a Contrast Ratio (CR value).

10. The method according to claim 1 or 2, wherein
the material is a glass ceramic.

11. The method according to claim 1 or 2, wherein
the manufacturing condition is a heat treatment condition.

12. The method according to claim 1 or 2, wherein
the material is a dental material.

13. A prediction system comprising:
an acquirer configured to acquire a composition and a manufacturing condition of a material of interest; and
a predictor configured to predict a plurality of properties of the material of interest by inputting the acquired composition and manufacturing condition of the material of interest into a machine learning model for predicting properties of a material based on its composition and manufacturing condition, wherein
the machine learning model is based on a mixture of continuous probability distributions.

14. A prediction system comprising:
an acquirer configured to acquire a plurality of properties of a material of interest; and
a predictor configured to predict a composition and a manufacturing condition of the material of interest based on the acquired properties of the material of interest by performing inverse analysis using a machine learning model for predicting properties of a material based on its composition and manufacturing condition, wherein
the machine learning model is based on a mixture of continuous probability distributions.
